# EUROPEAN PATENT SPECIFICATION

(11) **EP 3 366 283 B1**
(45) Date of publication and mention of the grant of the patent: **01.09.2021**
(21) Application number: 18165545.7
(22) Date of filing: 17.01.2005
(51) Int. Cl.: A61K 9/20, C07D 207/16, A61P 3/00, A61P 3/04, A61P 3/10, A61P 19/02, A61P 19/10, A61P 37/08, A61P 43/00

(54) **DIRECT COMPRESSION FORMULATION AND PROCESS**
DIREKTDRUCKFORMULIERUNG UND -VERFAHREN
FORMULATION DE COMPRESSION DIRECTE ET PROCÉDÉ

(30) Priority: 20.01.2004 US 537706 P; 25.08.2004 US 604274 P
(43) Date of publication of application: 29.08.2018
(62) Divisional of application: 15199440.7
(73) Proprietor: Novartis AG, 4056 Basel (CH)
(72) Inventor: KOWALSKI, James, East Hanover NJ 07936-1080 (US); LAKSHMAN, Jay Parthiban, Bridgewater, NJ 08807 (US); PATEL, Arun P., Succasunna, NJ 07876 (US)
(74) Representative: Carpmaels & Ransford LLP

(56) References cited:
- WO-A1-00/34241
- JP-A- 2003 327 532
- US-A1- 2003 078 247
- US-B1- 6 548 481
- VILLHAUER E B ET AL: "1-ÄÄ(3-HYDROXY-1-ADAMANTYL)AMINOÜACETYLÜ- 2-CYANO-(S)-PYRROLIDINE: A POTENT, SELECTIVE, AND ORALLY BIOAVAILABLE DIPEPTIDYL PEPTIDASE IV INHIBITOR WITH ANTIHYPERGLYCEMIC PROPERTIES", JOURNAL OF MEDICINAL CHEMISTRY, AMERICAN CHEMICAL SOCIETY, US, vol. 46, no. 13, 1 January 2003 (2003-01-01), pages 2774-2789, XP001165747, ISSN: 0022-2623, DOI: 10.1021/JM030091L

## Description

This invention relates to direct compressed pharmaceutical tablets comprising a dipeptidylpeptidase IV (DPP-IV) inhibitor compound which is (S)-1-[(3-hydroxy-1-adamantyl)amino]acetyl-2-cyanopyrrolidine.

DPP-IV inhibitors are known in the art. For example, DPP-IV inhibitors are in each case generically and specifically disclosed e.g. in WO 98/19998,DE19616 486 A1, WO 00/34241, WO 95/15309, WO 01/72290, WO 01/52825, WO 9310127, WO 9925719, WO 9938501, WO 9946272, WO 9967278 and WO 9967279.

In the direct compressed pharmaceutical tablet of the invention the DPP-IV inhibitor is (S)-1 - [(3-hydroxy-1 adamantyl)amino]acetyl-2-cyano-pyrrolidine.

Published patent application WO 0034241 and published patent US 6110949 disclose N-substituted adamantyl-amino-acetyl-2-cyano pyrrolidines and N-(substituted glycyl)-4-cyano pyrrolidines respectively. In particular these applications describe the DPP-IV inhibitor in the direct compressed pharmaceutical tablet of the invention which is the compound 1-[[(3-Hydroxy-1-adamantyl) amino]acetyl]-2-cyano-(S)-pyrrolidine (also known as LAF237).

DPP-IV inhibitor compounds are known and described in U.S. Patent No. 6,166,063, issued December 26, 2000 and WO 01/52825. Specially disclosed is (S)-1 -[(3-hydroxy-1 adamantyl)amino]acetyl-2-cyano-pyrrolidine (LAF237), which can exist in free form or in acid addition salt form. Pharmaceutically acceptable, i.e., non-toxic and physiologically acceptable, salts are preferred, although other salts are also useful, e.g., in isolating or purifying the compounds used in the direct compressed pharmaceutical tablet of this invention. Although the preferred acid addition salts are the hydrochlorides, salts of methanesulfonic, sulfuric, phosphoric, citric, lactic and acetic acid may also be utilized.

The DPP-IV inhibitor in the direct compressed pharmaceutical tablet of the invention is (S)-1-[(3-hydroxy-1-adamantyl)amino]acetyl-2-cyano-pyrrolidine (LAF237) of formula 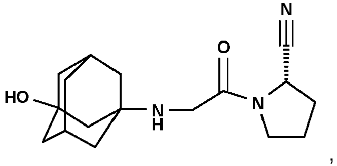 and optionally a pharmaceutical salt thereof.

LAF237 is specifically disclosed in Example 1 of WO 00/34241. LAF237 can be formulated as described on page 20 of WO 98/19998 or in WO 00/34241. The preferred formulations for the administration of LAF237 are described in the US provisional application No. 60/604274.

The DPP-IV inhibitor of the direct compressed pharmaceutical tablet of the invention and each of the examples, may be administered in enantiomerically pure form, e.g., >98%, preferably >99%; or together with the R enantiomer, e.g., in racemic form. The above dosage ranges are based on the compounds of formula (I), excluding the amount of the R enantiomer.

In view of its ability to inhibit DPP-IV, (S)-1-[(3-hydroxy-1-adamantyl)amino]acetyl-2-cyano-pyrrolidine and its corresponding pharmaceutically acceptable acid addition salts, are useful in treating conditions mediated by DPP-IV inhibition. Based on the above and findings in the literature, it is expected that the compound disclosed herein is useful in the treatment of conditions, such as non-insulin-dependent diabetes mellitus, arthritis, obesity, allograft transplantation and calcitonin-osteoporosis. In addition, based on the roles of glucagon-like peptides, such as GLP-1 and GLP-2, and their association with DPP-IV inhibition, it is expected that the compound disclosed herein is useful for example, to produce a sedative or anxiolytic effect, or to attenuate post-surgical catabolic changes and hormonal responses to stress, or to reduce mortality and morbidity after myocardial infarction, or in the treatment of conditions related to the above effects which may be mediated by GLP-1 and/or GLP-2 levels.

More specifically, the DPP-IV inhibitor compound (S)-1-[(3-hydroxy-1-adamantyl)amino]acetyl-2-cyano-pyrrolidine and its corresponding pharmaceutically acceptable acid addition salts, improve early insulin response to an oral glucose challenge and, therefore, are useful in treating non-insulin-dependent diabetes mellitus.

Certain DPP-IV inhibitor compounds are hygroscopic, present stability problems, and are not inherently compressible. Consequently, there is a need to provide a free-flowing and cohesive composition capable of being directly compressed into strong tablets with an acceptable *in vitro* dissolution profile. Tablets may be defined as solid dosage pharmaceutical forms containing drug substances with or without suitable fillers. They are produced by compression or compaction of a formulation containing the active ingredient and certain excipients selected to aid in the processing and to improve the properties of the product. Tablets may be coated or uncoated and are made from powdered, crystalline materials. They may include various diluents, binders, disintegrants, lubricants, glidants and in many cases, colorants. Excipients used are classified according to the function they perform. For example, a glidant may be used to improve the flow of powder blend in the hopper and into the tablet die.

There has been widespread use of tablets since the latter part of the 19^{th} century and the majority of pharmaceutical dosage forms are marketed as tablets. Major reasons of tablet popularity as a dosage form are simplicity, low cost and the speed of production. Other reasons include stability of drug product, convenience in packaging, shipping and dispensing. To the patient or consumer, tablets offer convenience of administration, ease of accurate dosage, compactness, portability, blandness of taste, ease of administration and elegant distinctive appearance.

Tablets may be plain, film or sugar coated bisected, embossed, layered or sustained-release. They can be made in a variety of sizes, shapes and colors. Tablets may be swallowed, chewed or dissolved in the buccal cavity or beneath the tongue. They may be dissolved in water for local or topical application. Sterile tablets are normally used for parenteral solutions and for implantation beneath the skin.

In addition to the active or therapeutic ingredients, tablets may contain a number of inert materials known as excipients. They may be classified according to the role they play in the final tablet. The primary composition includes a filler, binder, lubricant and glidant. Other excipients which give physical characteristics to the finished tablet are coloring agents, and flavors in the case of chewable tablets. Without excipients most drugs and pharmaceutical ingredients cannot be directly-compressed into tablets. This is primarily due to the poor flow and cohesive properties of most drugs. Typically, excipients are added to a formulation to impart good flow and compression characteristics to the material being compressed. Such properties are imparted to these excipients through pretreatment steps, such as wet granulation, slugging, spray drying spheronization or crystallization.

Lubricants are typically added to prevent the tableting materials from sticking to punches, minimize friction during tablet compression, and allow for removal of the compressed tablet from the die. Such lubricants are commonly included in the final tablet mix in amounts usually less than 1% by weight.

In addition, tablets often contain diluents which are added to increase the bulk weight of the blend resulting in a practical size for compression. This is often necessary where the dose of the drug is relatively small.

Another commonly used class of excipients in tablets is binders. Binders are agents, which impart cohesive qualities to the powdered material. Commonly used binders include starch, and sugars, such as sucrose, glucose, dextrose and lactose.

Disintegrants are often included to ensure that the tablet has an acceptable rate of disintegration. Typical disintegrants include starch derivatives and salts of carboxymethylcellulose.

Other desirable characteristics of excipients include the following:
- High-compressibility to allow strong tablets to be made at low compression forces;
- Good flow properties that can improve the flow of other excipients in the formula; and
- Cohesiveness (to prevent tablet from crumbling during processing, shipping and handling).

There are three commercially important processes for making compressed tablets: wet granulation, direct compression and dry granulation (slugging or roller compaction). The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets. Preformulation studies are done to determine the chemical and physical compatibility of the active component with proposed excipients.

The properties of the drug, its dosage forms and the economics of the operation will determine selection of the best process for tableting. Generally, both wet granulation and direct compression are used in developing a tablet.

The dry granulation method may be used where one of the constituents, either the drug or the diluent, has sufficient cohesive properties to be tabletted. The method consists of blending, slugging the ingredients, dry screening, lubrication and compression.

The wet granulation method is used to convert a powder mixture into granules having suitable flow and cohesive properties for tableting. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes weighing, dry powder blending, wet granulating, drying, milling, blending lubrication and compression.

In general, powders do not have sufficient adhesive or cohesive properties to form hard, strong granules. A binder is usually required to bond the powder particles together due to the poor cohesive properties of most powders. Heat and moisture sensitive drugs cannot usually be manufactured using wet granulation. The large number of processing steps and processing time are problems due to high level manufacturing costs. Wet granulation has also been known to reduce the compressibility of some pharmaceutical excipients, such as microcrystalline cellulose.

Direct compression is regarded as a relatively quick process where the powdered materials are compressed directly without changing the physical and chemical properties of the drug. The active ingredient(s), direct compression excipients and other auxiliary substances, such as a glidant and lubricant are blended in a twin shell blender or similar low shear apparatus before being compressed into tablets. This type of mixing was believed to be essential in order to prepare "pharmaceutically acceptable" dosage forms. Some pharmaceutical scientists believe that the manner in which a lubricant is added to a formulation must be carefully controlled. Accordingly, lubricants are usually added to a granulation by gentle mixing. It is also believed that prolonged blending of a lubricant with a granulation can materially affect hardness and disintegration time for the resulting tablets. Excessive blending of lubricants with the granulate ingredients can cause water proofing of the granule and reduces tablet hardness or strength of the compressed tablet. For these reasons, high-shear mixing conditions have not been used to prepare direct compression dosage forms.

The advantages of direct compression include uniformity of blend, few manufacturing steps involved, i.e., the overall process involves weighing of powders, blending and compression, hence less cost; elimination of heat and moisture, prime particle dissociation and physical stability.

Pharmaceutical manufacturers would prefer to use direct compression techniques over wet or dry granulation methods because of quick processing time and cost advantages. However, direct compression is usually limited to those situations where the drug or active ingredient has physical characteristics required to form pharmaceutically acceptable tablets. However, one or more excipients must often be combined with the active ingredient before the direct-compression method can be used since many ingredients do not have the necessary properties. Since each excipient added to the formulation increases the tablet size of the final product, manufacturers are often limited to using the direct-compression method in formulations containing a low dose of the active ingredient per compressed tablet.

A solid dosage form containing a high-dose drug, i.e., the drug itself comprises a substantial portion of the total compressed tablet weight, could only be directly compressed if the drug itself has sufficient physical characteristics, e.g., cohesiveness, for the ingredients to be directly compressed.

Certain DPP-IV inhibitors are considered high-dose drugs. Most tablet formulations include a range of 70-85% by weight of DPP-IV inhibitor per tablet. This high-dose drug, combined with its rather poor physical characteristics for direct compression, has not permitted direct compression as a method to prepare the final tablet. In addition, the active ingredients have poor stability in presence of water, another factor militating against the use of the wet granulation method.

Another limitation of direct compression as a method of tablet manufacturing is the potential size of the compressed tablets. If the amount of active ingredient is high, a pharmaceutical formulator may choose to wet granulate the active ingredient with other excipients to attain an acceptable sized tablet with the desired amount of active ingredient. The amount of filler, binder or other excipients needed in wet granulation is less than that required for direct compression since the process of wet granulation contributes toward the desired physical properties of the tablet.

Hydroxypropyl methylcellulose has been utilized in the pharmaceutical industry as a direct compression excipient for solid dose forms. Hydroxypropyl methylcellulose is a processed cellulose and controls drug release from solid dosage forms.

Despite the advantages of the direct compression, such as reduced processing time and cost, wet granulation is widely-used in the industry to prepare solid dosage forms. Wet granulation is often preferred over direct compression because wet granulation has a greater chance of overcoming any problems associated with the physical characteristics of various ingredients in the formulation. This provides material which has the required flow and cohesive properties necessary to obtain an acceptable solid dosage form.

The popularity of wet granulation compared to direct compression is based on at least three advantages. First, wet granulation provides the material to be compressed with better wetting properties, particularly in the case of hydrophobic drug substances. The addition of hydrophilic excipients makes the surface of the hydrophobic drug more hydrophilic, reducing disintegration and dissolution problems. Second, the content uniformity of the solid dosage form is generally improved with wet granulation because all of the granules usually contain the same amount of drug. Lastly, the segregation of drug(s) from excipients is avoided.

Segregation could be a potential problem with direct compression. The size and shape of particles comprising the granulate to be compressed are optimized through the wet granulation process. This is because when a dry solid is wet granulated the binder "glues" particles together, so that they agglomerate into spherical granules.

In spite of the advantages afforded by wet granulation in general, due to the instability of the compounds in the presence of water, it is desirable to directly compress tablets containing high-dose DPP-IV inhibitor, e.g. as that defined in formula (I). There is a need in the industry for techniques and pharmaceutical excipients which will allow manufacturers to prepare high-dose DPP-IV inhibitor tablets by direct compression.

It is an object of the invention to provide a direct compressed DPP-IV inhibitor tablet in unit dosage form having an acceptable dissolution profile, as well as acceptable degrees of hardness and resistance to chipping, as well as a short disintegration time.

In addition to the active ingredient, the direct compressed pharmaceutical tablet may contain a number of inert materials known as excipients. They may be classified according to the role they play in the final tablet. The primary composition includes fillers, binders or diluents, lubricants, disintegrants and glidants. Other excipients which give physical characteristics to the finished tablet are coloring agents, and flavors in the case of chewable tablets. Typically, excipients are added to a formulation to impart good flow and compression characteristics to the material being compressed.

The preferred formulation of this invention comprises the following: the active ingredient which is the DPP-IV inhibitor compound, the binders or diluents which are microcrystalline cellulose and lactose, the disintegrant which is sodium starch glycolate and the lubricant which is magnesium stearate.

One, two, three or more diluents can be selected. Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 15% to about 40% by weight of the composition. The preferred diluents include microcrystalline cellulose which is manufactured by the controlled hydrolysis of alpha-cellulose, obtained as a pulp from fibrous plant materials, with dilute mineral acid solutions. Following hydrolysis, the hydrocellulose is purified by filtration and the aqueous slurry is spray dried to form dry, porous particles of a broad size distribution. Suitable microcrystalline cellulose will have an average particle size of from about 20 nm to about 200 nm. Microcrystalline cellulose is available from several suppliers. Suitable microcrystalline cellulose includes Avicel PH 101, Avicel PH 102, Avicel PH 103, Avicel PH 105 and Avicel PH 200, manufactured by FMC Corporation. Particularly preferred in the practice of this invention is Avicel PH 102, which has the smallest surface area and pore structure. Preferably the microcrystalline cellulose is present in a tablet formulation in an amount of from about 25% to about 70% by weight. Another preferred range of this material is from about 30% to about 35% by weight; yet another preferred range of from about 30% to about 32% by weight.

Another diluent is lactose. Preferably, the lactose is ground to have an average particle size of between about 50 µm and about 500 µm prior to formulating. The lactose is present in the tablet formulation in an amount of from about 5% to about 40% by weight, and can be from about 18% to about 35% by weight, and most preferred, can be from about 20% to about 25% by weight.

One, two, three or more disintegrants can be selected. Examples of pharmaceutically acceptable disintegrants include, but are not limited to, starches; clays; celluloses; alginates; gums; cross-linked polymers, e.g., cross-linked polyvinyl pyrrolidone, cross-linked calcium carboxymethylcellulose and cross-linked sodium carboxymethylcellulose; soy polysaccharides; and guar gum. The disintegrant, e.g., may be present in an amount from about 2% to about 20%, e.g., from about 5% to about 10%, e.g., about 7% about by weight of the composition. A disintegrant is also an optional but useful component of the tablet formulation. Disintegrants are included to ensure that the tablet has an acceptable rate of disintegration. Typical disintegrants include starch derivatives and salts of carboxymethylcellulose. Sodium starch glycolate is the preferred disintegrant for this formulation. Preferably the disintegrant is present in the tablet formulation in an amount of from about 0% to about 10% by weight, and can be from about 1% to about 4% by weight, and most preferred, can be from about 1.5% to about 2.5% by weight.

One, two, three or more lubricants can be selected. Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose and microcrystalline cellulose. The lubricant, e.g., may be present in an amount from about 0.1% to about 5% by weight of the composition; whereas, the glidant, e.g., may be present in an amount from about 0.1% to about 10% by weight. Lubricants are typically added to prevent the tableting materials from sticking to punches, minimize friction during tablet compression and allow for removal of the compressed tablet from the die. Such lubricants are commonly included in the final tablet mix in amounts usually less than 1% by weight. The lubricant component may be hydrophobic or hydrophilic. Examples of such lubricants include stearic acid, talc and magnesium stearate. Magnesium stearate reduces the friction between the die wall and tablet mix during the compression and ejection of the tablets. It helps prevent adhesion of tablets to the punches and dies. Magnesium stearate also aids in the flow of the powder in the hopper and into the die. It has a particle size range of 450-550 microns and a density range of 1.00-1.80 g/mL. It is stable and does not polymerize within the tableting mix. The preferred lubricant, magnesium stearate is also employed in the formulation. Preferably, the lubricant is present in the tablet formulation in an amount of from about 0.25% to about 6%; also preferred is a level of about 0.5% to about 4% by weight; and most preferably from about 0.1% to about 2% by weight. Other possible lubricants include talc, polyethylene glycol, silica and hardened vegetable oils. In an optional embodiment of the invention, the lubricant is not present in the formulation, but is sprayed onto the dies or the punches rather than being added directly to the formulation.

Other conventional solid fillers or carriers, such as, cornstarch, calcium phosphate, calcium sulfate, calcium stearate, magnesium stearate, stearic acid, glyceryl mono- and distearate, sorbitol, mannitol, gelatin, natural or synthetic gums, such as carboxymethyl cellulose, methyl cellulose, alginate, dextran, acacia gum, karaya gum, locust bean gum, tragacanth and the like, diluents, binders, lubricants, disintegrators, coloring and flavoring agents could optionally be employed.

Examples of pharmaceutically acceptable binders include, but are not limited to, starches; celluloses and derivatives thereof, e.g., microcrystalline cellulose, hydroxypropyl cellulose hydroxylethyl cellulose and hydroxylpropylmethyl cellulose; sucrose; dextrose; corn syrup; polysaccharides; and gelatin. The binder, e.g., may be present in an amount from about 10% to about 40% by weight of the composition.

Additional examples of useful excipients are described in the Handbook of pharmaceutical excipients, 3rd edition , Edited by A.H.Kibbe, Published by: American Pharmaceutical Association, Washington DC, ISBN: 0-917330-96-X, or Handbook of Pharmaceutical Excipients (4th edition), Edited by Raymond C Rowe - Publisher: Science and Practice.

The invention is set out in the appended claims.

Thus, the present invention concerns a direct compressed pharmaceutical tablet, wherein the dispersion comprises particles comprising a DPP-IV inhibitor which is (S)-1-[(3-hydroxy-1-adamantyl)amino]acetyl-2-cyanopyrrolidine in free form or in acid addition salt form, and wherein;
i) at least 80% of the particle size distribution of the particles comprising (S)-1-[(3-hydroxy-1-adamantyl)amino]acetyl-2-cyanopyrrolidine in free form or in acid addition salt form in the tablet is between 10 µm and 250 µm and at least 35% of the particle size distribution of the particles comprising (S)-1-[(3-hydroxy-1-adamantyl)amino]acetyl-2-cyanopyrrolidine in free form or in acid addition salt form in the tablet is between 50 µm and 150 µm, as measured by laser diffraction;
ii) the water content of the tablet is less than 5% after 1 week at 25°C and 60% room humidity; and
iii) the tablet thickness to tablet weight ratio is from 0.002 to 0.06 mm/mg.

The weight of the DPP-IV inhibitor which is (S)-1-[(3-hydroxy-1-adamantyl)amino]acetyl-2-cyanopyrrolidine on a dry weight basis to tablet weight of diluent ratio may be of 0.5 to 0.25, preferably 0.4 to 0.28.

The direct compressed pharmaceutical tablet as described above, may further comprise diluents, wherein at least one diluent is a microcrystalline cellulose and wherein, the weight of DPP-IV inhibitor which is (S)-1-[(3-hydroxy-1-adamantyl)amino]acetyl-2-cyanopyrrolidine on a dry weight basis to tablet weight of microcrystalline cellulose ratio is of 2 to 0.333, preferably 1 to 0.333, most preferably of 0.7 to 0.333.

The direct compressed pharmaceutical tablet as described above may comprise between 20 and 120 mg of LAF237 preferably between 25 and 100m of LAF237 or a pharmaceutically acceptable acid addition salt thereof.

The direct compressed pharmaceutical tablet as described above may further comprise a diluent, wherein the diluent is selected from a microcrystalline cellulose and lactose, preferably microcrystalline cellulose and lactose are in the direct compressed pharmaceutical tablet.

The direct compressed pharmaceutical tablet as described above may comprise in addition;
(a) 0-20% by weight on a dry weight basis of a pharmaceutically acceptable disintegrant;
(b) 0.1-10% by weight on a dry weight basis of a pharmaceutically acceptable lubricant.

Preferably the direct compressed pharmaceutical tablet as described above comprises in addition;
(a) 1-6% by weight on a dry weight basis of a pharmaceutically acceptable disintegrant;
(b) 0.25-6% by weight on a dry weight basis of a pharmaceutically acceptable lubricant.

The above ratios have been obtained on a dry weight basis for the DPP-IV inhibitor and diluents.

In a further embodiment, the present invention concerns a direct compressed pharmaceutical tablet as defined by claim 1 comprising;
(a) 5-60% by weight on a dry weight basis of the DPP-IV inhibitor which is (S)-1-[(3-hydroxy-1-adamantyl)amino]acetyl-2-cyanopyrrolidine in free form or in acid addition salt form;
(b) 40-95% by weight on a dry weight basis of a pharmaceutically acceptable diluent;
(c) 0-20% by weight on a dry weight basis of a pharmaceutically acceptable disintegrant; and optionally
(d) 0.1-10% by weight on a dry weight basis of a pharmaceutically acceptable lubricant.

Preferably the present invention concerns a direct compressed pharmaceutical tablet as defined by claim 1 comprising;
(a) 20-40% by weight on a dry weight basis of the DPP-IV inhibitor which is (S)-1-[(3-hydroxy-1-adamantyl)amino]acetyl-2-cyanopyrrolidine in free form or in acid addition salt form;
(b) 40-95% by weight on a dry weight basis of a pharmaceutically acceptable diluent;
(c) 0-10% by weight on a dry weight basis of a pharmaceutically acceptable disintegrant; and optionally
(d) 0.25-6% by weight on a dry weight basis of a pharmaceutically acceptable lubricant.

Most preferably the present invention concerns a direct compressed pharmaceutical tablet as defined by claim 1 comprising;
(a) 20-35% by weight on a dry weight basis of the DPP-IV inhibitor which is (S)-1-[(3-hydroxy-1-adamantyl)amino]acetyl-2-cyanopyrrolidine in free form or in acid addition salt form;
(b) 40-95% by weight on a dry weight basis of a pharmaceutically acceptable diluent;
(c) 0-10% by weight on a dry weight basis of a pharmaceutically acceptable disintegrant; and optionally
(d) 0.25-6% by weight on a dry weight basis of a pharmaceutically acceptable lubricant.

Most preferably the present invention concerns a direct compressed pharmaceutical tablet as defined by claim 1 comprising;
(a) 20-35% by weight on a dry weight basis of the DPP-IV inhibitor which is (S)-1-[(3-hydroxy-1-adamantyl)amino]acetyl-2-cyanopyrrolidine in free form or in acid addition salt form;
(b) 62-78% by weight on a dry weight basis of a pharmaceutically acceptable diluent;
(c) 0-10% by weight on a dry weight basis of a pharmaceutically acceptable disintegrant; and optionally
(d) 0.1-10% by weight on a dry weight basis of a pharmaceutically acceptable lubricant.

Most preferably the present invention concerns a direct compressed pharmaceutical tablet as defined by claim 1 comprising;
(a) 20-35% by weight on a dry weight basis of a DPP-IV inhibitor which is (S)-1-[(3-hydroxy-1-adamantyl)amino]acetyl-2-cyanopyrrolidine in free form or in acid addition salt form;
(b) 62-78% by weight on a dry weight basis of a pharmaceutically acceptable diluent;
(c) 1-6% by weight on a dry weight basis of a pharmaceutically acceptable disintegrant; and optionally
(d) 0.25-6% by weight on a dry weight basis of a pharmaceutically acceptable lubricant.

Most preferably the present invention concerns a direct compressed pharmaceutical tablet as defined by claim 1 comprising;
(a) 22-28% by weight on a dry weight basis of the DPP-IV inhibitor which is (S)-1-[(3-hydroxy-1-adamantyl)amino]acetyl-2-cyanopyrrolidine in free form or in acid addition salt form;
(b) 66-76% by weight on a dry weight basis of a pharmaceutically acceptable diluent;
(c) 0-6% by weight on a dry weight basis of a pharmaceutically acceptable disintegrant; and optionally
(d) 0.25-6% by weight on a dry weight basis of a pharmaceutically acceptable lubricant.

Most preferably the present invention concerns a direct compressed pharmaceutical tablet as defined by claim 1 comprising;
(a) 22-28% by weight on a dry weight basis of the DPP-IV inhibitor which is (S)-1-[(3-hydroxy-1-adamantyl)amino]acetyl-2-cyanopyrrolidine in free form or in acid addition salt form;
(b) 66-76% by weight on a dry weight basis of a pharmaceutically acceptable diluent;
(c) 1-6% by weight on a dry weight basis of a pharmaceutically acceptable disintegrant; and optionally
(d) 0.25-6% by weight on a dry weight basis of a pharmaceutically acceptable lubricant.

In the present application the reference to a pharmaceutically acceptable diluent means at least one diluent, a mixture of e.g. 2 or 3 diluents is also covered.

Preferably the above described direct compressed pharmaceutical tablet comprises;
i) one or two diluents selected from microcrystalline cellulose and lactose
ii) the two diluents microcrystalline cellulose and lactose,
iii) 25-70% preferably 35-55% by weight on a dry weight basis of a pharmaceutically acceptable microcrystalline cellulose, or
iv) 25-70% preferably 35-55% by weight on a dry weight basis of a pharmaceutically acceptable microcrystalline cellulose and 5-40% preferably 18-35% of lactose.

Most preferably the above described direct compressed pharmaceutical tablets comprise one or two diluents selected from microcrystalline cellulose such as Avicel PH 102 and lactose.

Most preferably the direct compressed pharmaceutical tablets comprise the pharmaceutically acceptable lubricant (d).

In the present application the reference to a pharmaceutically acceptable disintegrant means at least one disintegrant, a mixture of e.g. 2 or 3 disintegrants is also covered.

In the present application the reference to a pharmaceutically acceptable lubricant means at least one lubricant, a mixture of e.g. 2 or 3 lubricants is also covered.

The DPP-IV inhibitor is LAF237 in free form or in acid addition salt form, preferred diluents are microcrystalline cellulose or lactose or preferably a combination of microcrystalline cellulose and lactose, preferred disintegrant is sodium starch glycolate, and preferred lubricant is magnesium stearate.

The particular components in the preferred direct compressed pharmaceutical tablet are the following:
(a) 20-35% by weight on a dry weight basis of DPP-IV inhibitor which is LAF237 in free form or in acid addition salt form;
(b) 25-70% by weight on a dry weight basis of a pharmaceutically acceptable microcrystalline cellulose;
(c) 5-40% by weight on a dry weight basis of a pharmaceutically acceptable lactose;
(d) 0-10% by weight on a dry weight basis of a pharmaceutically acceptable sodium starch glycolate;
(e) 0.25-6% by weight on a dry weight basis of magnesium stearate.

The particular components in the preferred composition of the direct compressed pharmaceutical tablet are the following:
(a) 25-35% by weight on a dry weight basis of DPP-IV inhibitor which is LAF237 in free form or in acid addition salt form;
(b) 25-70% by weight on a dry weight basis of a pharmaceutically acceptable microcrystalline cellulose;
(c) 5-40% by weight on a dry weight basis of a pharmaceutically acceptable lactose;
(d) 0-10% by weight on a dry weight basis of a pharmaceutically acceptable sodium starch glycolate;
(e) 0.25-6% by weight on a dry weight basis of magnesium stearate.

Another preferred composition of the direct compressed pharmaceutical tablet is the following:
(a) from about 30% to about 32% by weight on a dry weight basis of the DPP-IV inhibitor which is (S)-1-[(3-hydroxy-1-adamantyl)amino]acetyl-2-cyanopyrrolidine in free form or in acid addition salt form;
(b) from about 40% to about 45% by weight on a dry weight basis of a pharmaceutically acceptable microcrystalline cellulose;
(c) from about 20% to about 25% by weight on a dry weight basis of a pharmaceutically acceptable lactose;
(d) from about 1.5% to about 2.5% by weight on a dry weight basis of a pharmaceutically acceptable sodium starch glycolate; and
(e) from about 0.1% to about 2% by weight on a dry weight basis of magnesium stearate.

Another preferred composition of the direct compressed pharmaceutical tablet is the following:
(a) 20-35% preferably 22-28% by weight on a dry weight basis of the DPP-IV inhibitor which is LAF237 in free form or in acid addition salt form;
(b) 35-55% by weight on a dry weight basis of a pharmaceutically acceptable microcrystalline cellulose;
(c) 18-35% by weight on a dry weight basis of a pharmaceutically acceptable lactose;
(d) 1-4% by weight on a dry weight basis of a pharmaceutically acceptable sodium starch glycolate; and
(e) 0.5-4% by weight on a dry weight basis of magnesium stearate.

Still another preferred composition of the direct compressed pharmaceutical tablet is the following:
(a) from about 22% to about 28% preferably 24-26% by weight on a dry weight basis of the DPP-IV inhibitor which is (S)-1-[(3-hydroxy-1-adamantyl)amino]acetyl-2-cyanopyrrolidine in free form or in acid addition salt form;
(b) from about 45% to about 50% by weight on a dry weight basis of a pharmaceutically acceptable microcrystalline cellulose;
(c) from about 20% to about 25% by weight on a dry weight basis of a pharmaceutically acceptable lactose;
(d) from about 1.5% to about 2.5% by weight on a dry weight basis of a pharmaceutically acceptable sodium starch glycolate; and
(e) from about 0.1% to about 2% by weight on a dry weight basis of magnesium stearate.

Still another preferred composition of the direct compressed pharmaceutical tablet is the following:
(a) from 24-26% by weight on a dry weight basis of the DPP-IV inhibitor which is (S)-1-[(3-hydroxy-1-adamantyl)amino]acetyl-2-cyanopyrrolidine in free form or in acid addition salt form;
(b) from about 46% to about 48% by weight on a dry weight basis of a pharmaceutically acceptable microcrystalline cellulose;
(c) from about 23% to about 24.5% by weight on a dry weight basis of a pharmaceutically acceptable lactose;
(d) from about 1.5% to about 2.5% by weight on a dry weight basis of a pharmaceutically acceptable sodium starch glycolate; and
(e) from about 0.1% to about 2% by weight on a dry weight basis of magnesium stearate.

Still another preferred composition of the direct compressed pharmaceutical tablet is the following:
(a) 30-35% by weight on a dry weight basis of DPP-IV inhibitor which is LAF237 in free form or in acid addition salt form;
(b) 35-50% by weight on a dry weight basis of a pharmaceutically acceptable microcrystalline cellulose;
(c) 18-35% by weight on a dry weight basis of a pharmaceutically acceptable lactose;
(d) 1-4% by weight on a dry weight basis of a pharmaceutically acceptable sodium starch glycolate; and
(e) 0.5-4% by weight on a dry weight basis of magnesium stearate.

In a further embodiment, the present invention concerns any one of the above described compositions wherein the pharmaceutically acceptable lubricant (d) is only optionally comprised in the formulation. But preferably the pharmaceutically acceptable lubricant (d) is comprised in the composition.

Preferably for the direct compressed pharmaceutical tablets of the invention, the above described compositions comprise between 20 and 35% most preferably between 22 and 28% by weight on a dry weight basis of a DPP-IV inhibitor which is LAF237, in free form or in acid addition salt form.

Additional conventional excipients can optionally be added to the herein described formulations such as the conventional solid fillers or carriers described hereinabove.

The above described formulations are particularly adapted for the production of the direct compressed pharmaceutical tablets of the invention and provides the necessary physical characteristics, dissolution and drug release profiles as required by one of ordinary necessary physical skill in the art. Therefore, the present invention also concerns the use of any of the above described formulations, for the manufacture of pharmaceutical tablets in particular for direct compression.

In particular the direct compressed pharmaceutical tablets have very low friability problems, very good breaking strength, improved manufacturing robustness, optimal tablet thickness to tablet weight ratios (direct compressed tablets), less water in the formulation especially directed compressed tablet, good Dispersion Disintegration time DT according to the British Pharmacopoeia 1988, good Dispersion Quality.

This direct compressed tablets of the present invention comprising the DPP-IV inhibitor may be made by blending and compression. The choice of grades of excipients took into consideration particle size maintained within a range that allows homogeneity of the powder mix and content uniformity of DPP-IV inhibitor which is (S)-1-[(3-hydroxy-1-adamantyl)amino]acetyl-2-cyanopyrrolidine. It prevents segregation of powders in the hopper during direct compression. The advantages of using these excipients are that they impart compressibility, cohesiveness and flowability of the powder blend. In addition, the use of direct compression provides competitive unit production cost, shelf life, eliminates heat and moisture, allows for prime particle dissociation, physical stability and ensures particle size uniformity.

The described advantages of the claimed direct compressed pharmaceutical tablets are also very useful for e.g. roller compaction or wet granulation or to fill capsules.

The applicant has discovered that the direct compressed pharmaceutical tablet is particularly advantageous if;
i) at least 80% of the particle size distribution of the particles comprising (S)-1-[(3-hydroxy-1-adamantyl)amino]acetyl-2-cyanopyrrolidine in free form or in acid addition salt form in the tablet is between 10 µm and 250 µm and at least 35% of the particle size distribution of the particles comprising (S)-1-[(3-hydroxy-1-adamantyl)amino]acetyl-2-cyanopyrrolidine in free form or in acid addition salt form in the tablet is between 50 µm and 150 µm, as measured by laser diffraction;
ii) the water content of the tablet is less than 5% after 1 week at 25°C and 60% room humidity (RH); and
iii) tablet thickness to tablet weight ratio is from 0.002 to 0.06 mm/mg.

Thus, the present invention concerns a direct compressed pharmaceutical tablet as defined in the claims, comprising the DPP-IV inhibitor which is (S)-1-[(3-hydroxy-1-adamantyl)amino]acetyl-2-cyanopyrrolidine, in free form or in acid addition salt form, which has physical properties that render the tableting into direct compressed pharmaceutical tablet unlikely or very difficult.

Thus in a first embodiment (a), the present invention concerns direct compressed pharmaceutical tablets as defined in the claims, wherein the dispersion contains particles comprising DPP-IV inhibitor which is LAF237, in free form or in acid addition salt form, and wherein at least 80% and most preferably 90% of the particle size distribution of the particles comprising (S)-1-[(3-hydroxy-1-adamantyl)amino]acetyl-2-cyanopyrrolidine in free form or in acid addition salt form in the tablet is between 10 to 250 µm as measured by laser diffraction.

The term "wherein at least 80% and most preferably 90%" means at least 80% and most preferably at least 90%.

The term "wherein at least 35% and most preferably 45%" means at least 35% and most preferably at least 45%.

In particular, the present invention concerns direct compressed pharmaceutical tablets as defined in the claims, wherein the dispersion contains particles comprising the DPP-IV inhibitor, in free form or in acid addition salt form, and wherein at least 35% and most preferably 45% of the particle size distribution of the particles comprising (S)-1-[(3-hydroxy-1-adamantyl)amino]acetyl-2-cyanopyrrolidine in free form or in acid addition salt form in the tablet is between 50 to 150 µm as measured by laser diffraction.

In a second embodiment (b), this invention concerns a direct compressed pharmaceutical tablet as defined in the claims, wherein the dispersion contains particles comprising the DPP-IV inhibitor LAF237, in free form or in acid addition salt form, and wherein tablet thickness to tablet weight ratio is from 0.002 to 0.06 mm/mg, preferably from 0.01 to 0.03 mm/mg.

The combination of the above first and second embodiments (a) and (b), provide direct compressed tablets with good compaction characteristics.

Thus this invention concerns a direct compressed pharmaceutical tablet wherein the dispersion contains particles comprising the DPP-IV inhibitor which is (S)-1-[(3-hydroxy-1-adamantyl)amino]acetyl-2-cyanopyrrolidine, in free form or in acid addition salt form, and wherein;
i) at least 80% and most preferably 90% of the particle size distribution of the particles comprising (S)-1-[(3-hydroxy-1-adamantyl)amino]acetyl-2-cyanopyrrolidine in free form or in acid addition salt form in the tablet is between 10 to 250 µm as measured by laser diffraction;
ii) tablet thickness to tablet weight ratio is from 0.002 to 0.06 mm/mg or from 0.01 to 0.03 mm/mg;
iii) at least 35% and most preferably 45% of the particle size distribution of the particles comprising (S)-1-[(3-hydroxy-1-adamantyl)amino]acetyl-2-cyanopyrrolidine in free form or in acid addition salt form in the tablet is between 50 to 150 µm as measured by laser diffraction; and
iv) the water content of the tablet is less than 5% after 1 week at 25°C and 60% room humidity.

This invention concerns a direct compressed pharmaceutical tablet wherein the dispersion contains particles comprising the DPP-IV inhibitor which is (S)-1-[(3-hydroxy-1-adamantyl)amino]acetyl-2-cyanopyrrolidine, in free form or in acid addition salt form, and wherein;
i) at least 80% and most preferably 90% of the particle size distribution of the particles comprising (S)-1-[(3-hydroxy-1-adamantyl)amino]acetyl-2-cyanopyrrolidine in free form or in acid addition salt form in the tablet is between 10 to 250 µm and at least 35% of the particle size distribution of the particles comprising (S)-1-[(3-hydroxy-1-adamantyl)amino]acetyl-2-cyanopyrrolidine in free form or in acid addition salt form in the tablet is between 50 µm and 150 µm, as measured by laser diffraction,
ii) the water content of the tablet is less than 5% after 1 week at 25°C and 60% RH, and
iii) tablet thickness to tablet weight ratios is of 0.002 to 0.06 mm/mg.

Preferably this invention concerns a direct compressed pharmaceutical tablet, most preferably a direct compressed pharmaceutical tablet wherein the dispersion contains particles comprising the DPP-IV inhibitor which is LAF237, in free form or in acid addition salt form, and wherein;
i) at least 80% of the particle size distribution of the particles comprising (S)-1-[(3-hydroxy-1-adamantyl)amino]acetyl-2-cyanopyrrolidine in free form or in acid addition salt form in the tablet is between 10 µm and 250 µm and at least 35% and most preferably 45% of the particle size distribution of the particles comprising (S)-1-[(3-hydroxy-1-adamantyl)amino]acetyl-2-cyanopyrrolidine in free form or in acid addition salt form in the tablet is between 50 to 150 µm as measured by laser diffraction,
ii) the water content of the tablet is less than 5% after 1 week at 25°C and 60% RH, and
iii) tablet thickness to tablet weight ratios is of 0.01 to 0.03 mm/mg

In a very preferred aspect, the above described embodiments i.e. direct compressed pharmaceutical tablets contain;
(a) 20-35% by weight on a dry weight basis of a DPP-IV inhibitor in free form or in acid addition salt form, which is LAF237 in free form or in acid addition salt form;
(b) 40-95% by weight on a dry weight basis of a pharmaceutically acceptable diluent;
(c) 0-10% by weight on a dry weight basis of a pharmaceutically acceptable disintegrant;
(d) 0.25-6% by weight on a dry weight basis of a pharmaceutically acceptable lubricant.

Preferably the LAF237 particles comprise more than 70% of LAF237, most preferably more than 90% or 95% and even more preferably more than 98% of LAF237.

It has been discovered that the selected particle size distribution of LAF237 was particularly important to provide the best compaction of the tablets.

In an additional preferred embodiment, the particle size distribution of the selected excipients (b), (c) and/or (d) is similar to the particle size distribution of the LAF237 particles.

The term "similar", means that the particle size distribution of the excipient in the tablet is between 5 and 400µm, or between 10 and 300 µm, preferably between 10 to 250 µm.

The preferred excipients with an adapted particle size distribution can be picked from e.g. Handbook of Pharmaceutical Excipients (4th edition), Edited by Raymond C Rowe - Publisher: Science and Practice.

LAF237 particles size, is controlled by crystallisazion, drying and/or milling/sieving (non limiting examples are described below). Particle size can also be comminuted using roller compaction and milling/sieving. Producing the right particle size is well known and described in the art such as in "Pharmaceutical dosage forms: volume 2, 2nd edition, Ed.: H.A.Lieberman, L.Lachman, J.B.Schwartz (Chapter 3: SIZE REDUCTION)".

Multiple particle sizes have been studied and it has been discovered that the herein described specific size range provides unexpected good results for direct compaction. PARTICLE SIZE DISTRIBUTION ESTIMATION BY ANALYTICAL SIEVING: Particle size distribution can be measured using Sieve analysis, Photon Correlation Spectroscopy or laser diffraction (international standart ISO 13320-1), or electronic sensing zone, light obstruction, sedimentation or microscopy which are procedures well known by the person skilled in the art. According to the invention, particle size is determined by laser diffraction.

Sieving is one of the oldest methods of classifying powders by particle size distribution

Such methods are well known and described in the art such as in any analytical chemistry text book or by the United State Pharmacopeia's (USP) publication USP-NF (2004 - Chapter 786 - (The United States Pharmacopeial Convention, Inc., Rockville, MD)) which describes the US Food and Drug Administration (FDA) enforceable standards. The used techniques are e.g. described in Pharmaceutical dosage forms: volume 2, 2nd edition, Ed.: H.A.Lieberman, L.Lachman, J.B.Schwartz is a good example. It also mentions (page 187) additional methods: Electronic sensing zone, light obstruction, air permeation, sedimentation in gas or liquid.

In an air jet sieve measurement of particle size, air is drawn upwards, through a sieve, from a rotating slit so that material on the sieve is fluidised. At the same time a negative pressure is applied to the bottom of the sieve which removes fine particles to a collecting device. Size analyses and determination of average particle size are performed by removal of particles from the fine end of the size distribution by using single sieves consecutively. See also "Particle Size Measurement", 5th Ed. , p 178, vol. 1; T. Allen, Chapman & Hall, London, UK, 1997, for more details on this. For a person skilled in the art, the size measurement as such is thus of conventional character.

Water content of the tablet can be measured using Loss on drying method or Karl-Fischer method which are well known methods to the person skilled in the art (e.g. water content can be measured by loss on drying by thermogrametry). Such methods are well known and described in the art such as in any analytical chemistry text book (J.A. Dean, Analytical Chemistry Handbook, Section 19, McGraw-Hill, New York, 1995) or by the United State Pharmacopeia's (USP) publication USP-NF (2004) which describes the US Food and Drug Administration (FDA) enforceable standards ((2004 - USP - Chapter 921).

Tablet thickness is measurable using a ruler, vernier caliper, a screw gauge or any electronic method to measure dimensions. We take the tablet thickness in mm and divide by tablet weight in mg to get the ratio. Such methods are well known and described in the art such as in any analytical chemistry text book or by the United State Pharmacopeia's (USP) publication USP-NF (2004) which describes the US Food and Drug Administration (FDA) enforceable standards.

This invention provides in particular a compressed tablet or direct compressed tablet which is capable of dispersing in water within a period of 5 to 15 minutes to provide a dispersion which is capable of passing through a sieve screen with a mesh aperture of 710 µm in accordance with the herein defined British Pharmacopoeia test for dispersible tablets.

A tablet according to the invention, as well as being quickly dispersible in water, has the added advantage that it meets the British Pharmacopoeia (B.P.) test for dispersible tablets in respect of dispersion times and dispersion quality (i.e. passage through a 710 µ m sieve).

Preferably the dispersion time of a tablet according to the invention is less than 15 minutes, more preferably less than 12 minutes and most preferably less than 10 minute.

A further advantage of the tablets according to invention is that because a relatively fine dispersion is formed the tablet will have a lower dissolution time and thus the drug may be absorbed into the blood stream much faster. Furthermore the fast dispersion times and relatively fine dispersions obtained with tablets according to the invention are also advantageous for swallowable tablets. Thus tablets according to the invention can be presented both for dispersion in water and also for directly swallowing. Those tablets according to the invention that are intended for swelling are preferably film-coated to aid swallowing.

The present invention also concerns a direct compressed pharmaceutical tablet as defined in claim 1 with improved dissolution rates (dissolution of the drug), wherein the dispersion contains particles i.e. the DPPIV particles which are LAF237 particles comprising DPP-IV inhibitor which is LAF237, in free form or in acid addition salt form, wherein at least 80% and most preferably 90% of the particle size distribution of the particles comprising (S)-1-[(3-hydroxy-1-adamantyl)amino]acetyl-2-cyanopyrrolidine in free form or in acid addition salt form in the tablet is between 10 to 250 µm,
and wherein
i) between 0 and 10 minutes 85 to 99.5 % of the active ingredient is released, and
ii) between 10 and 15 minutes 90 to 99.5 % of the active ingredient is released,
preferably wherein,
i) between 0 and 10 minutes 88 to 99.5 % of the active ingredient is released, and
ii) between 10 and 15 minutes 95 to 99.5 % of the active ingredient is released,
or preferably
i) between 0 and 10 minutes 89 to 94 % of the active ingredient is released, and
ii) between 10 and 15 minutes 96 to 99 % of the active ingredient is released

The Paddle method to measure the drug dissolution rate (% of release) is used with 1000ml of 0.01N HCl. Such methods are well known and described in the art such as in any analytical chemistry text book or by the United State Pharmacopeia's (USP) publication USP-NF (2004 - Chapter 711) which describes the US Food and Drug Administration (FDA) enforceable standards.

Also described is a process for preparing a direct compressed DPP-IV inhibitor tablet in unit dosage form, wherein;
i) at least 80% and most preferably 90% of the particles comprising DPP-IV inhibitor which LAF237, in free form or in acid addition salt form, in the tablet have a particle size distribution of between 10 to 250 µm and at least 35% of the particle size distribution of the particles comprising LAF237 in free form or in acid addition salt form in the tablet is between 50 µm and 150 µm, as measured by laser diffraction,
ii) the water content of the tablet is less than 5% after 1 week at 25°C and 60% RH, and
iii) tablet thickness to tablet weight ratios is of 0.002 to 0.06 mm
which comprises:
(a) blending as a % by weight on a dry weight basis:
   (i) 5-60% by weight on a dry weight basis of the DPP-IV inhibitor which is LAF237 in free form or in acid addition salt form; and
   (ii) and at least one excipient selected from a diluent, a disintegrant and a lubricant,
   to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Preferably the above described process comprises:
(a) blending as a % by weight on a dry weight basis:
   (i) 5-60% by weight, on a dry weight basis of DPP-IV inhibitor which is LAF237 in free form or in acid addition salt form;
   (ii) 40-95% by weight on a dry weight basis of a pharmaceutically acceptable diluent;
   (iii) 0-20% by weight on a dry weight basis of a pharmaceutically acceptable disintegrant; and
   (iv) 0.1-10% by weight on a dry weight basis of a pharmaceutically acceptable lubricant,
   to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Most preferably the process comprises:
(a) blending as a % by weight on a dry weight basis:
   (i) 25-35% by weight on a dry weight basis of DPP-IV inhibitor which is LAF237 in free form or in acid addition salt form;
   (ii) 40-95% by weight on a dry weight basis of a pharmaceutically acceptable diluent;
   (iii) 0-10% by weight on a dry weight basis of a pharmaceutically acceptable disintegrant; and
   (iv) 0.25-6% by weight on a dry weight basis of a pharmaceutically acceptable lubricant,
   to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Preferably the blended composition used in step (a) is selected from the herein described preferred formulations.

The DPP-IV inhibitor is LAF237 in free form or in acid addition salt form, preferred diluents are microcrystalline cellulose or lactose or preferably a combination of microcrystalline cellulose and lactose, preferred disintegrant is sodium starch glycolate, and preferred lubricant is magnesium stearate.

In a best embodiment the process comprises:
(a) blending as a % by weight on a dry weight basis:
   (i) 20-35% or preferably 25-30% by weight by weight on a dry weight basis of DPP-IV inhibitor which is LAF237, in free form or in acid addition salt form;
   (ii) 25-70% by weight or preferably 35-50% by weight on a dry weight basis of a pharmaceutically acceptable microcrystalline cellulose such as Avicel PH 102;
   (iii) 5-40% by weight or preferably 18-35% by weight on a dry weight basis of a pharmaceutically acceptable lactose;
   (iv) 0-10% by weight or preferably 1-4% by weight on a dry weight basis of a pharmaceutically acceptable sodium starch glycolate; and
   (v) 0.25- 6% by weight or preferably 0.5-4% by weight on a dry weight basis of a pharmaceutically acceptable magnesium stearate.
      to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Also described is a process for preparing a direct compressed DPP-IV inhibitor tablet in unit dosage form which comprises:
(a) blending as a % by weight on a dry weight basis:
   (i) 30-32% by weight on a dry weight basis of DPP-IV inhibitor which is LAF237, in free form or in acid addition salt form;
   (ii) 40-45% by weight on a dry weight basis of a pharmaceutically acceptable microcrystalline cellulose (Avicel PH 102);
   (iii) 20-25% by weight on a dry weight basis of a pharmaceutically acceptable lactose;
   (iv) 1.5-2% by weight on a dry weight basis of a pharmaceutically acceptable sodium starch glycolate; and
   (v) 0.1-2% by weight on a dry weight basis of magnesium stearate,
      to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Also described is a process for preparing a direct compressed DPP-IV inhibitor tablet in unit dosage form which comprises:
(a) blending as a % by weight on a dry weight basis:
   (i) 23-28% by weight on a dry weight basis of DPP-IV inhibitor which is LAF237, in free form or in acid addition salt form;
   (ii) 45-50% by weight on a dry weight basis of a pharmaceutically acceptable microcrystalline cellulose (Avicel PH 102);
   (iii) 20-25% by weight on a dry weight basis of a pharmaceutically acceptable lactose;
   (iv) 1.5-2% by weight on a dry weight basis of a pharmaceutically acceptable sodium starch glycolate; and
   (v) 0.1-2% by weight on a dry weight basis of magnesium stearate,
      to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Before the compression step (b) a sieving step is preferably applied to the formulation for basic delumping i.e. to get rid of any agglomerates/cakes.

The DPP-IV inhibitor is 1-[3-hydroxy-adamant-1-ylamino)-acetyl]-pyrrolidine-2(S)-carbonitrile (LAF237 or vildagliptin).

In a further aspect, the present invention concerns the use of the herein described direct compressed tablets for the treatment of conditions, such as non-insulin-dependent diabetes mellitus, arthritis, obesity, allograft transplantation, calcitonin-osteoporosis, Heart Failure, Impaired Glucose Metabolism), IGT (Impaired Glucose Tolerance), neurodegenerative diseases such as Alzheimer's and Parkinson disease, modulating hyperlipidemia, modulating conditions associated with hyperlipidemia or for lowering VLDL, LDL and Lp(a) levels, cardiovascular or renal diseases e.g. diabetic cardiomyopathy, left or right ventricular hypertrophy, hypertrophic medial thickening in arteries and/or in large vessels, mesenteric vasculature hypertrophy, mesanglial hypertrophy, neurodegenerative disorders and cognitive disorders, to produce a sedative or anxiolytic effect, to attenuate post-surgical catabolic changes and hormonal responses to stress, to reduce mortality and morbidity after myocardial infarction, the treatment of conditions related to the above effects which may be mediated by GLP-1 and/or GLP-2 levels.

The following examples are not according to the claims:

### Example 1

To prepare the 25 mg tablet size (directly compressed tablet), a batch size of 7 kg is prepared using amounts corresponding to the following per unit: 25 mg per unit of the compound 1-[3-hydroxy-adamant-1-ylamino)-acetyl]-pyrrolidine-2(S)-carbonitrile is mixed with 35.1 mg of microcrystalline cellulose, 17.5 mg anhydrous lactose and 1.6 mg sodium starch glycolate. The ingredients are pre-blended together in a commercial bin blender, then sieved through a 500 µm or 850 µm screen. The mix is blended again in the bin blender, then the necessary amount of the magnesium stearate to yield the 0.8 mg magnesium stearate per 25 mg tablet size, is added. Blending in each step is conducted at about 150-450 rotations, to ensure homogeneity of the mixture. Following blending again in the bin blender, the mix can be tabletted in a conventional tableting machine. The individual tablet weight for the 25 mg tablet is 80 mg. Tablets having 50 mg active ingredient weigh 160 mg, and 100 mg active ingredient tablets weigh 320 mg, respectively. The blend is a powder which has excellent compressibility into the desired tablet size.

### Example 2

The same process as described above in example 1, can be applied to produce the below described preferred 50 mg tablet (directly compressed).

| **Components** | **Composition per unit (mg)** | **Quantity per batch (kg)** |
|---|---|---|
| LAF 237 drug substance | 50.00 | 65.0 |
| Microcrystalline cellulose, PH102 (Ph.Eur., NF) | 95.68 | 124.38 |
| Lactose anhydrous DT (USP, Ph.Eur.) | 47.82 | 62.17 |
| Sodium starch glycolate (USP, Ph.Eur.) | 4.00 | 5.2 |
| Magnesium stearate (Ph.Eur, NF) | 2.50 | 3.25 |
| Total weight, per tablet or per batch | 200.0 | 260.0 |

**Example 3:** The tablets prepared in accordance with the above Description and examples can be tested as follows.

### Tablet Evaluation Methods

1. Average tablet weight. Twenty tablets are weighed on an analytical balance and the average tablet weight calculated.
2. Tablet breaking strength (kilo bond-kp). 5 tablets are individually tested using a Schleuniger crushing strength tester, and the average breaking strength calculated.
3. Friability (% loss). 10 tablets, accurately weighed, are subjected to 10 minutes friability testing using a Roche Friabilator. The tablets are dedusted, reweighed, and the weight loss due to the friability is calculated as a percentage of the initial weight.
4. Dispersion Disintegration time DT (The test for dispersible tablets defined in the British Pharmacopoeia, 1988, Volume II, page 895 - BP 1988). 6 tablets are tested in accordance to the above-defined BP test (without discs) for dispersible tablets. This utilizes water at a temperature of 19°- 21°C.
5. Dispersion Quality. In accordance with the BP uniformity of dispersion test for dispersible tablets (BP 1988 Volume II page 895), two tablets are placed in 100 ml of water at 19°-21° C. and allowed to disperse.

### Granule Evaluation Methods

1. Loss on Drying (LOD). The residual moisture content of the granule (LOD) can be determined on a 3-4 g sample using a Computrac moisture analyser set at 90° C. operated in accordance with the manufacturer's procedure.
2. Weight Median Diameter (WMD). A 10 g sample of granule is sifted for 2 minutes at suitable pulse and sift amplitudes in an Allen Bradley sonic sifter in accordance with manufacturer's instructions. Sieves of 300 µm, 250 µm, 200 µm, 150 µm, 100 µm, 53 µm and 40 µm are used. The WMD is calculated from the cumulative percentage undersize size distribution using a computer program.

### Example 4:

### Improved manufacturing robustness

A preliminary compactibility assessment is carried out on a Carver press using different formulations as well as binary mixtures of LAF 237 with different excipients e.g. microcrystalline cellulose (Avicel PH102).

Data demonstrate that our claimed compositions on being compressed with increasing levels of pressure (compression force) show a substantially useful increase in tablet strength. In particular e.g. mixture of LAF237 and Avicel show a substantially useful increase in tablet strength. These results indicated that from compactibility point of view microcrystalline cellulose e.g. Avicel would a preferred excipient to be combined with LAF237. With increasing pressure (compression force) our claimed formulations and selected ranges show a substantially useful increase in tablet strength.

A compactibility study (D. Becker, personal communication) is carried out on an instrumented Korsch single station press with force and displacement sensors on both upper and lower punches.

A clear indication is afforded from these data that LAF237 tablets are very likely to have poor tablet hardness/crushing strength unless diluted out using sufficient filler with excellent compactibility. Our claimed formulations and selected ranges are particularly adapted to provide the required compactibility. Microcrystalline cellulose e.g. Avicel is a good choice for a filler in this respect.

### Example 5: Friability

Evaluation is carried out using a Manesty Betapress at 6 different settings: strain rate settings of 66-90 rpm (63,000-86,000 TPH) and force of 7.5-15 kN. The trials uses Flat-faced Beveled-edge (FFBE) tooling of 9 mm diameter for 250 mg tablets and 10 mm diameter for 310 mg tablets (other diameters are used depending on the weight of the tested tablet) . Total tablet weights were selected so that both the 9 and 10 mm FFBE tablets would have 100 mg of LAF237 and identical tablet thickness. Friability, Compression profile, Strain rate profile and Weight variation are the measured outcomes. Study design and the friability results obtained from the study are used to determine the variables (particle size distribution in the formulation, tablet weight, tablet thickness and weight, water content in the tablet etc) impacting the outcome of hardness.

### Example 6: Mechanical stress (particle size distribution)

The material in the desired particle size range can be produced from any form of vildagliptin e.g. amorphous vildagliptin, by mechanical stress. This stress can be mediated by impact, shear or compression. In most commercially available grinding equipment a combination of these principles occurs. For vildagliptin preferably a mechanical impact or jet mill is used. The most preferable mechanical impact mill can be equipped with different kind of beaters, screens, liners or with pin plates. For our process preferably an impact mill with plate beater and a slit screen 5 * 2.5 cm is used. The impact speed should be variable between 20 and 100 m/s (as peripheral speed) to adapt to any batch to batch variation. In our case a peripheral speed of the beater of about 40 - 50 m/s is used.

## Claims

1. A direct compressed pharmaceutical tablet, wherein the dispersion comprises particles comprising a DPP-IV inhibitor which is (S)-1-[(3-hydroxy-1-adamantyl)amino]acetyl-2-cyanopyrrolidine in free form or in acid addition salt form, and wherein
i) at least 80% of the particle size distribution of the particles comprising (S)-1-[(3-hydroxy-1-adamantyl)amino]acetyl-2-cyanopyrrolidine in free form or in acid addition salt form in the tablet is between 10 µm and 250 µm and at least 35% of the particle size distribution of the particles comprising (S)-1-[(3-hydroxy-1-adamantyl)amino]acetyl-2-cyanopyrrolidine in free form or in acid addition salt form in the tablet is between 50 µm and 150 µm, as measured by laser diffraction;
ii) the water content of the tablet is less than 5% after 1 week at 25°C and 60% room humidity; and
iii) the tablet thickness to tablet weight ratio is from 0.002 to 0.06 mm/mg.

2. The direct compressed pharmaceutical tablet of claim 1, wherein the tablet comprises between 20mg and 120mg of (S)-1-[(3-hydroxy-1-adamantyl)amino]acetyl-2-cyanopyrrolidine or a pharmaceutically acceptable acid addition salt thereof.

3. The direct compressed pharmaceutical tablet of claim 1, wherein the tablet comprises between 25mg and 100mg of (S)-1-[(3-hydroxy-1-adamantyl)amino]acetyl-2-cyanopyrrolidine or a pharmaceutically acceptable acid addition salt thereof.

4. The direct compressed pharmaceutical tablet of any preceding claim comprising (S)-1-[(3-hydroxy-1-adamantyl)amino]acetyl-2-cyanopyrrolidine and a pharmaceutically acceptable diluent, wherein the weight of (S)-1-[(3-hydroxy-1-adamantyl)amino]acetyl-2-cyanopyrrolidine on a dry weight basis to tablet weight of diluent ratio is from 0.5 to 0.25.

5. The direct compressed pharmaceutical tablet of claim 4, wherein the weight of (S)-1-[(3-hydroxy-1-adamantyl)amino]acetyl-2-cyanopyrrolidine on a dry weight basis to tablet weight of diluent ratio is from 0.4 to 0.28.

6. The direct compressed pharmaceutical tablet of any preceding claim, wherein the tablet thickness to weight ratio is from 0.01 to 0.03 mm/mg.

7. The direct compressed pharmaceutical tablet of any preceding claim, wherein the tablet comprises (S)-1-[(3-hydroxy-1-adamantyl)amino]acetyl-2-cyanopyrrolidine in free form or in acid addition salt form, microcrystalline cellulose, lactose, sodium starch glycolate and magnesium stearate.

8. The direct compressed pharmaceutical tablet of any preceding claim, wherein the tablet comprises microcrystalline cellulose.

## Patentansprüche

1. Direkt komprimierte pharmazeutische Tablette, wobei die Dispersion Partikel umfasst, die einen DPP-IV-Inhibitor umfassen, bei dem es sich um (S)-1-[(3-Hydroxy-1-adamantyl)amino]acetyl-2-cyanopyrrolidin in freier Form oder in Säureadditionssalzform handelt, und wobei
i) mindestens 80% der Teilchengrößenverteilung der (S)-1-[(3-Hydroxy-1-adamantyl)amino]acetyl-2-cyanopyrrolidin in freier Form oder in Säureadditionssalzform umfassenden Partikel in der Tablette zwischen 10 µm und 250 µm ist und mindestens 35% der Teilchengrößenverteilung der (S)-1-[(3-Hydroxy-1-adamantyl)amino]acetyl-2-cyanopyrrolidin in freier Form oder in Säureadditionssalzform umfassenden Partikel in der Tablette zwischen 50 µm und 150 µm ist, gemessen durch Laserdiffraktion,
ii) der Wassergehalt der Tablette nach 1 Woche bei 25°C und 60% Raumfeuchtigkeit weniger als 5% beträgt und
iii) das Verhältnis von Tablettendicke zu Tablettengewicht 0,002 bis 0,06 mm/mg beträgt.

2. Direkt komprimierte pharmazeutische Tablette nach Anspruch 1, wobei die Tablette zwischen 20 mg und 120 mg (S)-1-[(3-Hydroxy-1-adamantyl)amino]acetyl-2-cyanopyrrolidin oder eines pharmazeutisch unbedenklichen Säureadditionssalzes davon umfasst.

3. Direkt komprimierte pharmazeutische Tablette nach Anspruch 1, wobei die Tablette zwischen 25 mg und 100 mg (S)-1-[(3-Hydroxy-1-adamantyl)amino]acetyl-2-cyanopyrrolidin oder eines pharmazeutisch unbedenklichen Säureadditionssalzes davon umfasst.

4. Direkt komprimierte pharmazeutische Tablette nach einem der vorhergehenden Ansprüche, umfassend (S)-1-[(3-Hydroxy-1-adamantyl)amino]acetyl-2-cyanopyrrolidin und ein pharmazeutisch unbedenkliches Verdünnungsmittel, wobei das Verhältnis vom Gewicht des (S)-1-[(3-Hydroxy-1-adamantyl)amino]acetyl-2-cyanopyrrolidin auf einer Trockengewichtsbasis zum Tablettengewicht des Verdünnungsmittels 0,5 bis 0,25 beträgt.

5. Direkt komprimierte pharmazeutische Tablette nach Anspruch 4, wobei das Verhältnis vom Gewicht von (S)-1-[(3-Hydroxy-1-adamantyl)amino]acetyl-2-cyanopyrrolidin auf einer Trockengewichtsbasis zum Tablettengewicht des Verdünnungsmittels 0,4 bis 0,28 beträgt.

6. Direkt komprimierte pharmazeutische Tablette nach einem der vorhergehenden Ansprüche, wobei das Verhältnis von Tablettendicke zum Gewicht 0,01 bis 0,03 mm/mg beträgt.

7. Direkt komprimierte pharmazeutische Tablette nach einem der vorhergehenden Ansprüche, wobei die Tablette (S)-1-[(3-Hydroxy-1-adamantyl)amino]acetyl-2-cyanopyrrolidin in freier Form oder in Säureadditionssalzform, mikrokristalline Cellulose, Lactose, Natriumstärkeglykolat und Magnesiumstearat umfasst.

8. Direkt komprimierte pharmazeutische Tablette nach einem der vorhergehenden Ansprüche, wobei die Tablette mikrokristalline Cellulose umfasst.

## Revendications

1. Pastille pharmaceutique comprimée directe, la dispersion comprenant des particules comprenant un inhibiteur de DPP-IV qui est la (S)-1-[(3-hydroxy-1-adamantyl)amino]acétyl-2-cyanopyrrolidine sous forme libre ou sous forme de sel d'addition d'acide, et
i) au moins 80 % de la distribution de taille de particules des particules comprenant de la (S)-1-[(3-hydroxy-1-adamantyl)amino]acétyl-2-cyanopyrrolidine sous forme libre ou sous forme de sel d'addition d'acide dans la pastille étant entre 10 µm et 250 µm et au moins 35 % de la distribution de taille de particules des particules comprenant de la (S)-1-[(3-hydroxy-1-adamantyl)amino]acétyl-2-cyanopyrrolidine sous forme libre ou sous forme de sel d'addition d'acide dans la pastille étant entre 50 µm et 150 µm, tel que mesuré par diffraction laser ;
ii) la teneur en eau de la pastille étant inférieure à 5 % après 1 semaine à 25 °C et 60 % d'humidité ambiante ; et
iii) le rapport d'épaisseur de pastille sur poids de pastille étant de 0,002 à 0,06 mm/mg.

2. Pastille pharmaceutique comprimée directe selon la revendication 1, la pastille comprenant entre 20 mg et 120 mg de (S)-1-[(3-hydroxy-1-adamantyl)amino]acétyl-2-cyanopyrrolidine ou d'un sel d'addition d'acide pharmaceutiquement acceptable correspondant.

3. Pastille pharmaceutique comprimée directe selon la revendication 1, la pastille comprenant entre 25 mg et 100 mg de (S)-1-[(3-hydroxy-1-adamantyl)amino]acétyl-2-cyanopyrrolidine ou d'un sel d'addition d'acide pharmaceutiquement acceptable correspondant.

4. Pastille pharmaceutique comprimée directe selon une quelconque revendication précédente comprenant de la (S)-1-[(3-hydroxy-1-adamantyl)amino]acétyl-2-cyanopyrrolidine et un diluant pharmaceutiquement acceptable, le rapport du poids de (S)-1-[(3-hydroxy-1-adamantyl)amino]acétyl-2-cyanopyrrolidine sur une base de poids sec sur le poids de diluant de pastille étant de 0,5 à 0,25.

5. Pastille pharmaceutique comprimée directe selon la revendication 4, le rapport du poids de (S)-1-[(3-hydroxy-1-adamantyl)amino]acétyl-2-cyanopyrrolidine sur une base de poids sec sur le poids de diluant de pastille étant de 0,4 à 0,28.

6. Pastille pharmaceutique comprimée directe selon une quelconque revendication précédente, le rapport d'épaisseur sur poids de pastille étant de 0,01 à 0,03 mm/mg.

7. Pastille pharmaceutique comprimée directe selon une quelconque revendication précédente, la pastille comprenant de la (S)-1-[(3-hydroxy-1-adamantyl)amino]acétyl-2-cyanopyrrolidine sous forme libre ou sous forme de sel d'addition d'acide, une cellulose microcristalline, du lactose, du glycolate d'amidon sodique et du stéarate de magnésium.

8. Pastille pharmaceutique comprimée directe selon une quelconque revendication précédente, la pastille comprenant une cellulose microcristalline.
